# EUROPEAN PATENT SPECIFICATION

(11) **EP 1 970 029 B1**
(45) Date of publication and mention of the grant of the patent: **23.11.2011**
(21) Application number: 08250760.9
(22) Date of filing: 06.03.2008
(51) Int. Cl.: A61F 2/30, A61F 2/36

(54) **Improvements to implant**
Verbesserungen für Implantate
Améliorations pour implant

(30) Priority: 09.03.2007 GB 0704537
(43) Date of publication of application: 17.09.2008
(73) Proprietor: Orthosurgical SA, 11471 Athens (GR)
(72) Inventor: Michelinakis, Emmanuel, 11471 Athens (GR); Michelinakis, Mathios, 11471 Athens (GR)
(74) Representative: Wood, Graham

(56) References cited:
- EP-A- 0 128 036
- FR-A- 2 639 821
- FR-A- 2 668 058
- FR-A- 2 676 914
- FR-A- 2 683 717

## Description

The invention to which this application relates is to an improvement in the form of a stem implant which is for use, typically as part of a hip replacement system.

In any hip replacement operation and in subsequent use of the hip replacement, there are several factors which need to be taken into account. One of these is the longevity of the implant once it has been fitted and also the comfort of the patient to whom the implant has been fitted and the ability for the hip replacement to be used in natural range of motion. In the initial stages, it is important that there is provided a strong fixation of the implant in position in the patient. It is found that if a strong fixation can be achieved initially then this improves the chances of long term stability of the implant and as a result the improved quality of life for the patient can be achieved.

The use of stem implants is well known and generally known to comprise a neck or spigot, leading to an elongate member with a distal tip. The elongate member is fitted into the femur bone and the neck or spigot receives a femoral head component thereon. There are many different designs and variations of the implant which are the subject of many differing prior art patents. However, even with these different designs, problems are still experienced in both the initial fixation and long term stability of the implants in the patient. These problems, in the worst cases, can cause considerable pain and discomfort to the patient, can cause failure of the implant itself and/or require further surgery to be performed for the removal of the prosthesis. These requirements add further expense to the medical system and cause significant distress to the patient.

The stem is implanted directly into the femur bone press-fit without the use of cement.. Fixation is defined as the stable mechanical joining of the femoral component to the femur such that no relative motion occurs between the implant and the bone at the fixation interface. Tapered uncemented femoral components rely on initial three-point fixation followed by proximal porous bone ingrowth or ongrowth for continued stability. The points of fixation are achieved by the implant at two spaced points on the posterior side of the elongate member or stem and at an intermediate location on the anterior side.

The features of the preamble of claim 1 are know from document FR-A-2676914.

Document FR2683717 discloses an implant which includes an implant with a surface finish which is created by the application of material thereto and grooves formed in the part of the implant to which the material has been applied.

An aim of the present invention is to provide a stem implant devise in a form which provides improved fixation characteristics when initially fitted in the patient, and, as a result, improved stability and longevity of the hip implant.

To a first aspect of the invention, there is provided an implant, said implant having, at a first end, a neck formation, leading to an elongate member, the distal end of which defines the opposing end of the implant, said implant having an intermediate portion between the neck formation and the distal end, said intermediate portion having a surface finish which is created by the application of a coating material thereto, said implant further including first and second groups of rib members positioned on opposing sides of the elongate member, characterised in that said groups of rib members are exclusively located between the said intermediate portion and the said distal end.

Typically, the surface finish of the said portion is provided by applying a porous coating thereto. The porous coating is provided to encourage bone in-growth onto the implant and thus provides improved fixation and long term stability.

In one embodiment, the porous coating is achieved by applying titanium beads to promote the growth of the host bone onto the surface of the implant. Preferably, the coating is applied around the circumference of the said intermediate, also referred to as the proximal part of the stem portion so that the whole of the portion has a surface finish formed by the porous coating.

In one embodiment the area of the implant between the said porous coated intermediate part and the distal end is grit blasted to encourage bone attachment and on-growth thereon. In one embodiment the distal end has a tip which is polished.

In one embodiment, the longitudinal axes of the rib members are substantially parallel with the longitudinal axis of the elongate member. Typically the rib members in each group are spaced apart along the circumference of the elongate member

Typically each rib member in each group has a first end in line with the first end of each of the other rib members in the group but the respective lengths of the rib members vary depending on the position of the rib member in the group.

In one embodiment the height of the top of the rib member from the implant surface decreases from a first location at the end of the rib closest to the neck, to the opposing and. Typically the decrease in height is continuous along the length of said rib. In one embodiment the width of each rib remains constant along part of the length of the rib and from which point the width of the rib decreases towards a point at the end of the rib closest to the distal end of the elongate member.

Typically the longitudinal axis of each of the ribs in each group remain substantially parallel.

Typically, the implant is manufactured from titanium and more preferably, Titanium 6 Aluminium 4 Vanadium (Ti6A14V) alloy Thus, typically, with the use of the titanium beads coating in the intermediate portion the external surface of the implant is formed of titanium.

Typically, the elongate member, from the intermediate portion to the distal end, is tapered and preferably, is tapered by 3 decrees and typically the tapering is in the three planes of coronal, sagittal and transverse. This allows the elongate member to match anatomical characteristics of the femur bone and is found to prevent stress and provides correct anatomical loading distribution throughout the femur hence reducing distal fixation which may cause thigh pain in the patient to which the implant is fitted.

Furthermore, the taper design of the component acts as a wedge to physically lock the implant into place, proximally, and to prevent the implant from sinking deeper into the femur than is required. The wedge shape provides a frictionally stable implant in the femur enhancing the long term stability of the prosthesis..

In one embodiment the area of the elongate member between the said portion and the distal end is grit blasted to encourage rough surface finish to encourage bone on growth.

In a further embodiment the tip at the distal end is polished.

In one embodiment the implant is formed, typically by forging, of titanium.

A specific embodiment of the invention is now described with preference to the accompanying drawings wherein;

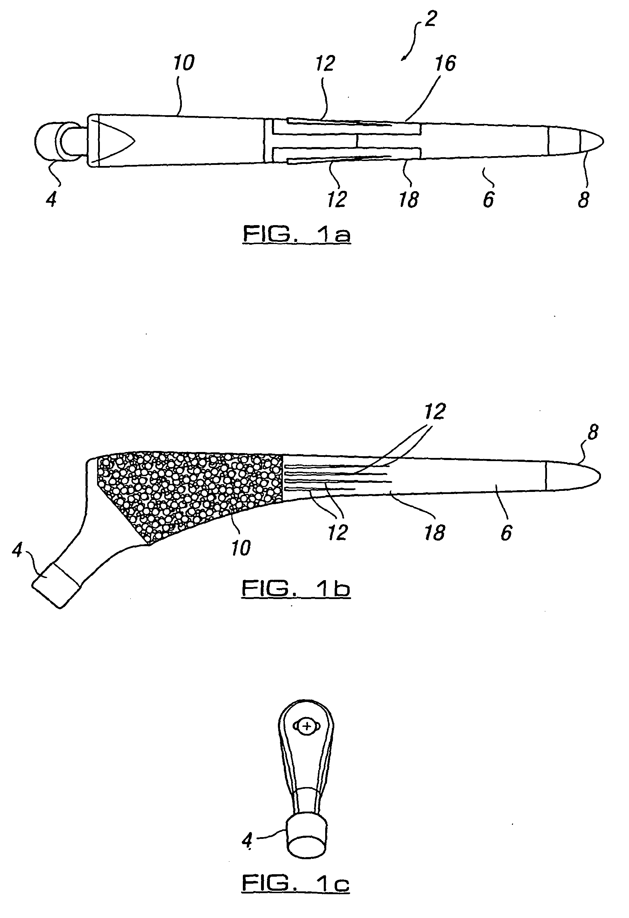

Figures 1a-c illustrate a plan, elevation and side views of a stem implant in accordance with one embodiment of the invention;
Figures 2a-p illustrate cross sectional views of the implant of Figure 1;
Figures 3a- o illustrate cross sectional views of the implant of Figure 1 but of different parts than shown in Figures 2a-p;
Figure 4 illustrates a cross sectional view along line F-F of Figure 2b, showing one half of the implant and ribs in greater detail; and
Figure 5 illustrates an embodiment of an implant in accordance with the invention.

Referring to the drawings there is shown a stem implant 2 of a design in accordance with one embodiment of the invention. The implant comprises a neck portion 4 leading to an elongate member section 6 with a distal tip 8. The neck portion 4 includes a part which is provided to receive a head thereon which is not shown but which can be of any conventional form. Following from the neck portion there is provided an intermediate portion 10 which can also be referred to as the proximal of a stem portion. In accordance with a preferred embodiment of the invention, and as shown, this intermediate portion 10 is coated with a porous material. It is also coated around the entire intermediate portion. In the embodiment shown the coating which is applied is sintered titanium beads and the pore size is, in one embodiment, in the range of 80-259µm. In one embodiment the microsphere porosity is in the range of 35-40%. It is found that the provision of this coating improves the ongrowth of bone once the implant has been fitted in position and, as a result improves the stability and strength of the implant when in position.

The elongate member 8 is also provided with a plurality of rib members 12. The rib members are provided in a specific configuration which has been found to improve the fixation of the stem implant in the femur and in particular prevent rotation of the implant. The elongate member also tapers inwardly towards the distal tip 8 at a taper of 3 degrees which is found to match closely with the taper of the femur and hence improve the comfort of the patient with the implant fitted.

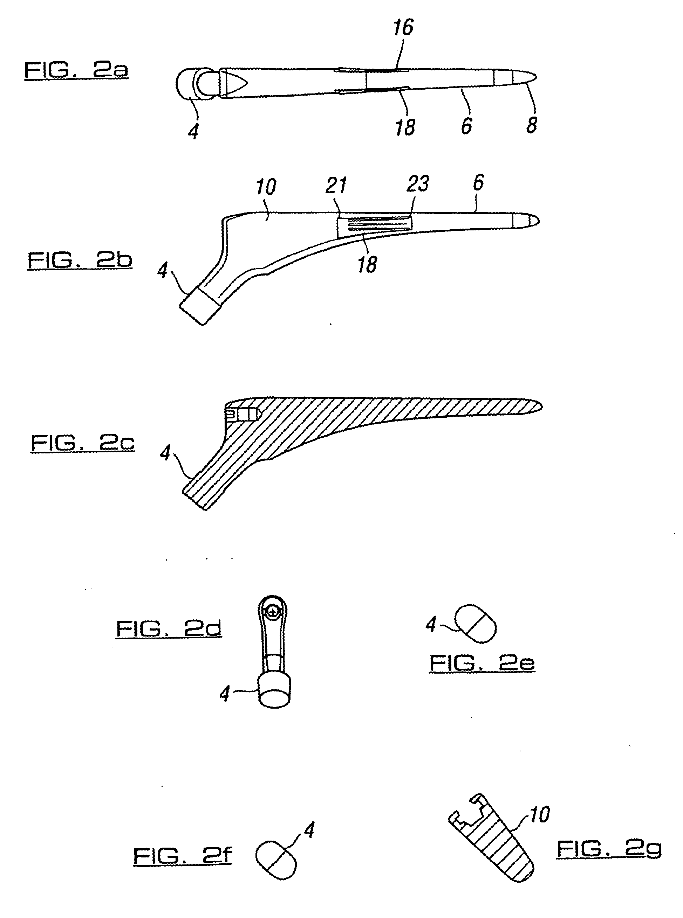

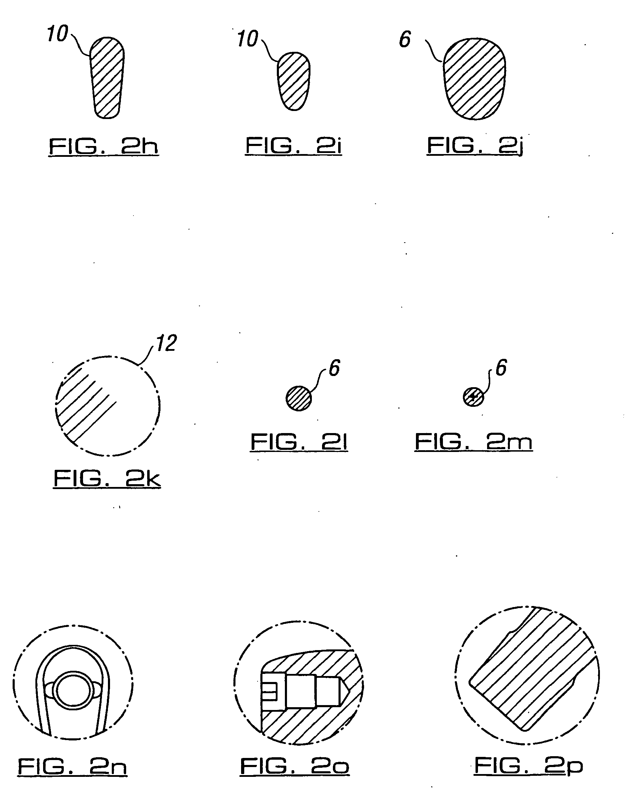

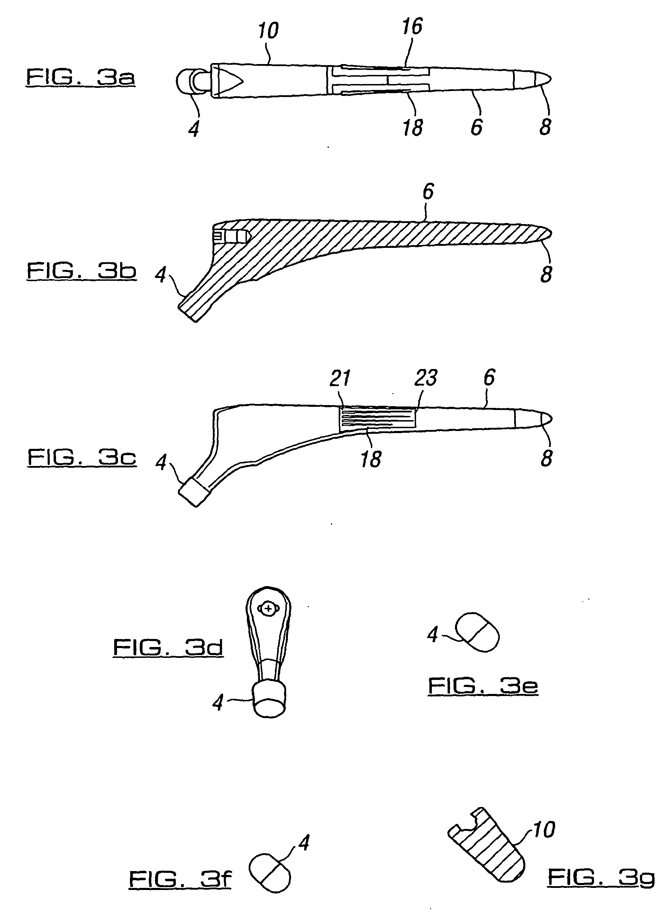

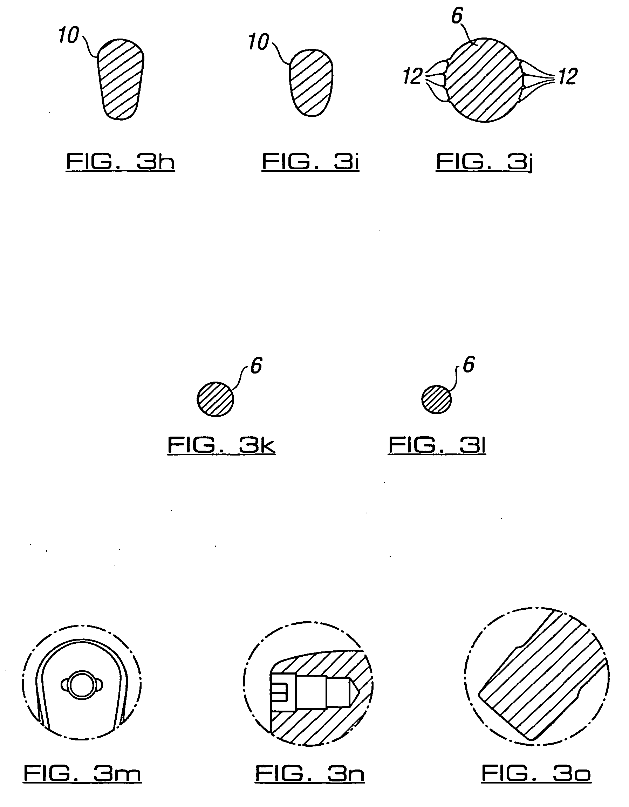

It will be seen from Figures 2a-p and 3a-o that the rib members 12 are provided in two groups 16, 18 and neither group extends along the length to the distal end, which is contrary to other conventional stem implants. Instead, the ribs extend from the intermediate coated portion towards the distal tip for a length of approximately a third to half of the length of the elongate member. Each rib narrows in the direction of the distal tip and tapers to a point. In one embodiment the spacing between the centres of adjacent ribs in each group is 2.7 mm.

In Figures 2a-p the embodiment shown has three ribs provided in each of the groups on opposing sides of the implant. In Figures 3a-o four rib members are provided in each group.

In whichever embodiment, typically each of the ribs decreases in height from the rib end 21 towards the rib end 23. While the ribs may be of different lengths, typically in each case the decrease in height for each particular rib will be continuous. The width of each of the ribs is preferably kept constant for a substantial length of the same until at a location along the length, intermediate the ends, the rib width is narrowed such that at the end 23 the rib narrows to a point.

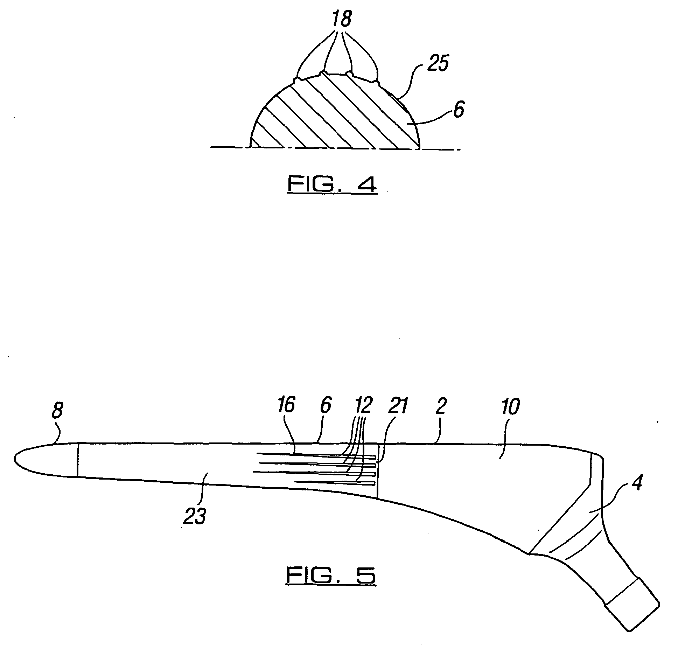

Figure 4 shows a cross section through one half of the implant along the line FF of Figure 2b, of larger scale. It shows how each of the ribs in the group 18 are spaced apart by a set distance and located so as to lie along the shaped outer periphery wall of the implant 6.

Figure 5 illustrates one embodiment of an implant in accordance with the invention. The provision of one of groups of rib members can be seen with, in this embodiment four rib members, of varying lengths being provided in the group. Also clearly shown is the intermediate portion with a surface finish created by the application of the beads thereto. The beads are can be applied to the required location on the implant, possibly using a mixture of the beads with adhesive or resin medium. The implant and beads are then heated to a sufficient temperature to allow the same to bond to form the coating. The area of the elongate member from the said portion towards the distal tip and in which the rib members are located, is grit blasted to form a roughened surface in comparison to the polished distal tip.

The provision of the ribs and the placing of the same in groups in conjunction with the roughened surface finish created by grit blasting the area of the implant in which the rib members are located is found to improve the fixation of the stem implant in position. This, in conjunction with the improved bone ongrowth which is achieved by coating the entire surface of the intermediate portion 10, means that the length of the elongate member or stem formed in accordance with the invention can be significantly reduced in comparison to conventional stem implants as the required level of anchoring of the implant in position can be achieved with the implant in accordance with the invention. The reduction in length means that the distal tip does not have to be placed as far into the patient's femur and, therefore, there isn't any possibility of bone formation due to mechanical stress, distally, which may cause distal fixation of the implant, and thigh pain. It also reduces the surgical invasive work required and in turn reduces discomfort to the patient.

## Claims

1. An implant (2), said implant having, at a first end, a neck formation (4), leading to an elongate member (6), the distal end (8) of which defines the opposing end of the implant, said implant having an intermediate portion (10) between the neck formation and the distal end, said intermediate portion (10) having a surface finish which is created by the application of a coating material thereto, said implant further including first and second groups (16,18) of rib members (12) positioned on opposing sides of the elongate member, **characterised in that** said groups of rib members are exclusively located between the said intermediate portion and the said distal end.

2. An implant according to claim 1 **characterised in that** the surface finish of the intermediate portion is provided by applying a porous coating material thereto.

3. An implant according to claim 2 **characterised in that** the porous coating is provided to encourage bone growth into the implant.

4. An implant according to claim 1 **characterised in that** the porous coating material includes titanium beads.

5. An implant according to claim 1 **characterised in that** the coating is applied around the circumference of the said intermediate portion so that the whole of the portion has a surface finish formed by the porous coating.

6. An implant according to claim 1 **characterised in that** the longitudinal axes of the rib members are substantially parallel with the longitudinal axis of the elongate member.

7. An implant according to claim 6 where the rib members in each group are spaced apart about the circumference of the elongate member.

8. An implant according to claim 1 **characterised in that** each rib member in each group has a first end in line with the first end of each of The other rib members in the group but the respective lengths of the rib members in the group varies.

9. An implant according to claim 8 **characterised in that** the height of the rib members in each group decrease from the rib member closest to the neck, to the rib member at the opposing end of the group.

10. An implant according to claim 9 **characterised in that** the decrease in height is continuous in said direction away from the first location.

11. An implant according to claim 10 **characterised in that** the width of each rib member is substantially constant along part of the length of the rib member and from which point, the width of the rib decreases towards the point at the end of the rib member closest to the distal end of the elongate member.

12. An implant according to claim 1 **characterised in that** the longitudinal axes of each of the ribs in each group remain substantially parallel.

13. An implant according to claim 1 **characterised in that** the implant is manufactured from titanium.

14. An implant according to claim 13 **characterised in that** the external surface of the implant, with the exception of the intermediate portion, is formed of titanium.

15. An implant according to claim 14 **characterised in that** the said surface is polished.

16. An implant according to claim 1 **characterised in that** the elongate member, from the intermediate portion to the distal end is tapered.

17. An implant according to claim 16 **characterised in that** the tapering is in three planes of coronal, sagittal and transverse.

18. An implant according to claim 16 wherein the taper angle is 3 degrees.

19. An implant according to claim 1 **characterised in that** the external surface of the intermediate portion is formed of Titanium beads.

20. An implant according to claim 1 **characterised in that** the external surface of the portion between the intermediate and the distal end is grit blasted to form a relatively rough surface finish.

## Patentansprüche

1. Implantat (2), wobei das genannte Implantat an einem ersten Ende ein Halsgebilde (4) hat, das zu einem länglichen Element (6) führt, dessen distales Ende (8) das entgegengesetzte Ende des Implantats definiert, wobei das genannte Implantat einen Zwischenabschnitt (10) zwischen dem Halsgebilde und dem distalen Ende hat, wobei der genannte Zwischenabschnitt (10) eine Oberflächenbeschaffenheit hat, die durch das Auftragen eines Beschichtungsmaterials auf ihn geschaffen wurde, wobei das Implantat ferner eine erste und eine zweite Gruppe (16, 18) von Rippenelementen (12) beinhaltet, die an entgegengesetzten Seiten des länglichen Elements positioniert sind, **dadurch gekennzeichnet, dass** sich die genannten Gruppen von Rippenelementen ausschließlich zwischen dem genannten Zwischenabschnitt und dem genannten distalen Ende befinden.

2. Implantat nach Anspruch 1, **dadurch gekennzeichnet, dass** die Oberflächenbeschaffenheit des Zwischenabschnitts durch Auftragen eines porösen Beschichtungsmaterials auf ihn bereitgestellt wurde.

3. Implantat nach Anspruch 2, **dadurch gekennzeichnet, dass** die poröse Beschichtung zum Fördern von Knochenwachstum in das Implantat bereitgestellt ist.

4. Implantat nach Anspruch 1, **dadurch gekennzeichnet, dass** das poröse Beschichtungsmaterial Titankugeln beinhaltet.

5. Implantat nach Anspruch 1, **dadurch gekennzeichnet, dass** die Beschichtung um den Umfang des genannten Zwischenabschnitts aufgetragen wird, so dass der gesamte Abschnitt eine von der porösen Beschichtung gebildete Oberflächenbeschaffenheit hat.

6. Implantat nach Anspruch 1, **dadurch gekennzeichnet, dass** die Längsachsen der Rippenelemente im Wesentlichen parallel zur Längsachse des länglichen Elements sind.

7. Implantat nach Anspruch 6, wobei die Rippenelemente in jeder Gruppe um den Umfang des länglichen Elements voneinander beabstandet sind.

8. Implantat nach Anspruch 1, **dadurch gekennzeichnet, dass** jedes Rippenelement in jeder Gruppe ein erstes Ende hat, das mit dem ersten Ende jedes der anderen Rippenelemente in der Gruppe auf gleicher Linie liegt, aber die jeweilige Länge der Rippenelemente in der Gruppe variiert.

9. Implantat nach Anspruch 8, **dadurch gekennzeichnet, dass** die Höhe der Rippenelemente in jeder Gruppe von dem Rippenelement, das dem Hals am nächsten ist, zu dem Rippenelement am entgegengesetzten Ende der Gruppe abnimmt.

10. Implantat nach Anspruch 9, **dadurch gekennzeichnet, dass** die Abnahme der Höhe in der genannten Richtung von dem ersten Ort weg kontinuierlich ist.

11. Implantat nach Anspruch 10, **dadurch gekennzeichnet, dass** die Breite jedes Rippenelements entlang einem Teil der Länge des Rippenelements im Wesentlichen konstant ist und die Breite der Rippe von diesem Punkt an in Richtung auf den Punkt am Ende des Rippenelements, das dem distalen Ende des Längselements am nächsten ist, abnimmt.

12. Implantat nach Anspruch 1, **dadurch gekennzeichnet, dass** die Längsachsen jeder der Rippen in jeder Gruppe im Wesentlichen parallel bleiben.

13. Implantat nach Anspruch 1, **dadurch gekennzeichnet, dass** das Implantat aus Titan hergestellt ist.

14. Implantat nach Anspruch 13, **dadurch gekennzeichnet, dass** die Außenfläche des Implantats mit Ausnahme des Zwischenabschnitts aus Titan gebildet ist.

15. Implantat nach Anspruch 14, **dadurch gekennzeichnet, dass** die genannte Oberfläche poliert ist.

16. Implantat nach Anspruch 1, **dadurch gekennzeichnet, dass** sich das längliche Element vom Zwischenabschnitt bis zum distalen Ende verjüngt.

17. Implantat nach Anspruch 16, **dadurch gekennzeichnet, dass** die Verjüngung in drei Ebenen, der Frontal-, Sagittal- und Transversalebene, erfolgt.

18. Implantat nach Anspruch 16, wobei der Verjüngungswinkel 3 Grad beträgt.

19. Implantat nach Anspruch 1, **dadurch gekennzeichnet, dass** die Außenfläche des Zwischenabschnitts aus Titankugeln gebildet ist.

20. Implantat nach Anspruch 1, **dadurch gekennzeichnet, dass** die Außenfläche des Abschnitts zwischen dem Zwischen- und dem distalen Ende gestrahlt ist, um eine relativ raue Oberflächenbeschaffenheit zu bilden.

## Revendications

1. Implant (2), ledit implant ayant, à sa première extrémité, une formation en col (4), menant à une partie allongée (6), dont l'extrémité distale (8) définit l'extrémité opposée de l'implant, ledit implant ayant une portion intermédiaire (10) entre la formation en col et l'extrémité distale, ladite portion intermédiaire (10) ayant une finition de surface qui est créée par application d'un matériau de revêtement à celle-ci, ledit implant comportant en outre un premier et un second groupe (16,18) de nervures (12) **caractérisés en ce que** lesdits groupes de nervures sont disposés sur les côtés opposés de la partie allongée et situés exclusivement entre ladite portion intermédiaire et ladite extrémité distale.

2. Implant selon la revendication 1 **caractérisé en ce que** la finition de surface de la portion intermédiaire est obtenue en appliquant un matériau de revêtement poreux à celle-ci.

3. Implant selon la revendication 2 **caractérisé en ce que** le revêtement poreux est fourni afin d'encourager la croissance osseuse dans l'implant.

4. Implant selon la revendication 1 **caractérisé en ce que** le matériau de revêtement poreux comprend des billes de titane.

5. Implant selon la revendication 1 **caractérisé en ce que** le revêtement est appliqué autour de la circonférence de ladite portion intermédiaire afin que la totalité de la portion ait une finition de surface formée par le revêtement poreux.

6. Implant selon la revendication 1 **caractérisé en ce que** les axes longitudinaux des nervures sont essentiellement parallèles à l'axe longitudinal de la partie allongée.

7. Implant selon la revendication 6, les nervures dans chaque groupe étant espacées sur la circonférence de la partie allongée.

8. Implant selon la revendication 1 **caractérisé en ce que** chaque nervure dans chaque groupe a une première extrémité alignée avec la première extrémité de chacune des autres nervures dans le groupe, mais les longueurs respectives des nervures dans le groupe varient.

9. Implant selon la revendication 8 **caractérisé en ce que** la hauteur des nervures dans chaque groupe diminue depuis la nervure la plus proche du col, jusqu'à-la nervure à l'extrémité opposée du groupe.

10. Implant selon la revendication 9 **caractérisé en ce que** la diminution de hauteur est continue dans ladite direction s'éloignant du premier emplacement.

11. Implant selon la revendication 10 **caractérisé en ce que** la largeur de chaque nervure est essentiellement constante le long d'une partie de la longueur de la nervure et à partir d'un point, la largeur de la nervure diminue vers le point à l'extrémité de la nervure la plus proche de l'extrémité distale de la partie allongée.

12. Implant selon la revendication 1 **caractérisé en ce que** les axes longitudinaux de chacune des nervures dans chaque groupe restent essentiellement parallèles.

13. Implant selon la revendication 1 **caractérisé en ce que** l'implant est fabriqué en titane.

14. Implant selon la revendication 13 **caractérisé en ce que** la surface externe de l'implant, à l'exception de la portion intermédiaire, est formée de titane.

15. Implant selon la revendication 14 **caractérisé en ce que** ladite surface est polie.

16. Implant selon la revendication 1 **caractérisé en ce que** la partie allongée, de la portion intermédiaire à l'extrémité distale est effilée.

17. Implant selon la revendication 16 **caractérisé en ce que** le rétrécissement est selon trois plans, coronal, sagittal et transversal.

18. Implant selon la revendication 16 **caractérisé en ce que** l'angle de rétrécissement est de 3 degrés.

19. Implant selon la revendication 1 **caractérisé en ce que** la surface externe de la portion intermédiaire est formée de billes de titane.

20. Implant selon la revendication 1 **caractérisé en ce que** la surface externe de la partie entre la portion intermédiaire et l'extrémité distale est grenaillée pour former une finition de surface relativement rugueuse.
